# EUROPÄISCHE PATENTSCHRIFT

(11) **EP 4 044 919 B1**
(45) Veröffentlichungstag und Bekanntmachung des Hinweises auf die Patenterteilung: **25.10.2023**
(21) Anmeldenummer: 20797400.7
(22) Anmeldetag: 15.10.2020
(51) Int. Cl.: A61B 5/11

(54) **INDIVIDUALISIERTE STURZPRÄVENTION**
INDIVIDUALIZED FALL PREVENTION
PRÉVENTION DE CHUTE INDIVIDUALISÉE

(30) Priorität: 18.10.2019 DE 102019128162
(43) Veröffentlichungstag der Anmeldung: 24.08.2022
(73) Patentinhaber: DizzyCure GmbH, 14195 Berlin (DE)
(72) Erfinder: BASTA, Dietmar, 14656 Brieselang (DE); ERNST, Arneborg, 14169 Berlin (DE)
(74) Vertreter: Hertin und Partner Rechts- und Patentanwälte PartG mbB
(86) Internationale Anmeldenummer: PCT/EP2020/079043
(87) Internationale Veröffentlichungsnummer: WO 2021/074295

(56) Entgegenhaltungen:
- US-A1- 2005 067 816
- US-A1- 2011 230 791
- US-A1- 2016 038 061
- US-A1- 2017 352 240
- US-A1- 2018 000 385
- US-A1- 2018 228 404

## Beschreibung

Die Erfindung betrifft eine Vorrichtung mit mindestens einem Sensor zur Messung der Veränderung einer Körperposition und/oder eines Bewegungsmusters des Trägers. Beim Überschreiten einer Stabilitätsgrenze wird ein Feedbacksignal ausgegeben. Die Stabilitätsgrenze kann ausgehend von einer Grundeinstellung auf Basis von Messdaten über die Körperbewegung und Eingabedaten (z.B. Sturz ja/nein) kontinuierlich aktualisiert werden. Zentraler Bestandteil der Vorrichtung ist die Regelungseinheit, bei der alle Eingabedaten zusammengeführt werden und die über mindestens einen Signalgeber aufgrund vorliegender und gespeicherter Daten das Feedbacksignal erzeugt.

### Hintergrund und Stand der Technik

Im Gleichgewichtssystem des Menschen ist die Lage des Körperschwerpunktes die geregelte Größe. Dafür werden multimodale Informationen verarbeitet. Diese werden z. B. in den propriozeptiven, somatosensorischen, visuellen und auditorischen Eingängen rezipiert. Die gesamte Verarbeitung der sensorischen Informationen und die Aktivierung der entsprechenden motorischen Efferenzen dient letztendlich dazu, den Körperschwerpunkt unter verschiedenen sensomotorischen Konditionen in einem engen Bereich zu halten. Wird das realisiert, stürzt der Mensch nicht. Bei Defiziten hinsichtlich der sensorischen Inputs, der zentralnervösen Signalverarbeitung oder bei der Auslösung der motorischen Stellreaktionen erhöht sich das Sturzrisiko. Patienten entwickeln dann meist ein mit Schwindelsymptomen verbundenes Unsicherheitsgefühl. Durch ein spezifisches Gleichgewichtstraining kann oft ein Funktionsverlust kompensiert und damit das Sturzrisiko verringert werden. Meist ist es dennoch gegenüber Gesunden erhöht. Bei älteren Menschen ab dem 60. Lebensjahr treten oben genannte Defizite zudem häufiger auf und werden aufgrund der abnehmenden zentralnervösen Plastizität im Alter weniger gut kompensiert. Das verbleibende Sturzrisiko ist dann besonders hoch. Hinzu kommt, dass gerade im Alter die Stellreflexe oft verlangsamt sind und Stürze gehäuft und mit größerer Schwere stattfinden. Es ist jedoch seit vielen Jahren bekannt, dass die zentralnervöse Verarbeitung der multimodalen sensorischen Informationen im Gleichgewichtssystem hinsichtlich der Verarbeitungsgeschwindigkeit und der Effektorintensität wesentlich von der aktuellen Aufmerksamkeit bzw. Vigilanz abhängt. So wird jeder Gleichgewichtsreflex bei hoher Vigilanz schneller und stärker ausgeführt als bei geringer Vigilanz. Eine hohe Vigilanz kann mithilfe einer kontinuierlichen Aktivierung aufrechterhalten bzw. durch geeignete plötzliche Reize erzeugt werden. Wird die Erhöhung der Vigilanz durch plötzliche Reize im richtigen Zeitpunkt erreicht, können stabilisierende Bewegungen zur Sturzverhinderung eingeleitet werden. Dabei ist es besonders bedeutsam, dass diese plötzlichen Reize nur bei der Abwendung eines Sturzereignisses zur Anwendung kommen, da bei zu häufiger Reizdarbietung eine Gewöhnung einsetzt und somit die gewünschte Erhöhung der Vigilanz ausbleibt. Somit soll der Reiz nur dann ausgelöst werden, wenn die Verlagerung des Körperschwerpunktes einen für die Stabilität der Körperhaltung kritischen Wert erreicht. Dieser Wert ist sehr individuell, je nach Krankheitsbild, Alter und allgemeinem Fitnesszustand einer Person.

Im Stand der Technik existieren Methoden zur Detektion eines fast-Sturzereignisses. Weiss et al. BMC Research Notes 3:62 (2010) beschreibt die Erkennung von fast-Sturzereignissen anhand von 3-D Beschleunigungsmustern, die an der Hüfte mithilfe von Accelerometern gemessen wurden.

WO002010150260A1 beschreibt eine Methode zur Detektion von fast-Sturzereignissen anhand der Verwendung von Accelerometer- und Gyrometerdaten in mathematischen Modellen und dem Abgleich der Ergebnisse mit vorgegebenen Schwellen. Im Stand der Technik wird jedoch von einer Laborsituation ausgegangen in der die hohe Individualität der Körperstabilität infolge von Erkrankungen und/oder Alterung keine Berücksichtigung findet. Zudem wird beim Überschreiten eines Schwellenwerts der fast-Sturzdetektion kein Feedbacksignal initiiert um einen Sturz abzuwenden.

Die Verwendung eines Feedbacksignals (im weiteren Sinne) zur Sturzprävention wird in WO002016078158A1 offenbart. Es wird jedoch nicht auf die wichtige Auswahl der Modalität des Feedbacksignals eingegangen. Außerdem wird die Feedbackschwelle nicht individualisiert ermittelt, was für eine sturzverhindernde Funktion essenziell ist.

US-2017/352240-A1, US-2011/230791-A1 und US-2018/228404-A1 zeigen bekannte Methoden zur Sturzprävention. US-2016/038061-A1, US-2018/000385-A1 und US-2005/067816-A1 beschreiben Verfahren zur Sturzdetektion.

### Aufgabe der Erfindung

Aufgabe der Erfindung ist es, eine Vorrichtung zur Sturzprävention ohne die Nachteile des Standes bereitzustellen. Insbesondere war es eine Aufgabe der Erfindung, eine Vorrichtung für eine sichere, lernfähige, individualisierte und effiziente Sturzprävention bereitzustellen, die sich einem Nutzer und seinem Bewegungsmuster anpasst, eine hohe Compliance aufweist und die Vigilanz des Nutzers bewahrt.

### Zusammenfassung der Erfindung

Gelöst wird die Aufgabe durch die Merkmale der unabhängigen Ansprüche. Vorteilhafte Ausgestaltungen der Erfindung sind in den abhängigen Ansprüchen beschrieben.

In einem ersten Aspekt betrifft die Erfindung eine Vorrichtung zur Sturzprävention nach Anspruch 1.

Die Vorrichtung umfasst mindestens einen Sensor zur Messung der Veränderung einer Körperposition und/oder eines Bewegungsmusters des Trägers. Beim Überschreiten einer Stabilitätsgrenze wird ein Feedbacksignal ausgegeben. Die Stabilitätsgrenze kann ausgehend von einer Grundeinstellung auf Basis von Messdaten über die Körperbewegung und Eingabedaten (z.B. Sturz ja/nein) kontinuierlich aktualisiert werden. Zentraler Bestandteil der Vorrichtung ist die Regelungseinheit, bei der alle Eingabedaten zusammengeführt werden und die über mindestens einen Signalgeber aufgrund vorliegender und gespeicherter Daten das Feedbacksignal erzeugt.

Eine Vorrichtung zur Sturzprävention beschreibt eine Vorrichtung, die einem Nutzer vor Stürzen aufgrund eines Verlustes des Gleichgewichts bewahrt. Ein Verlust des Gleichgewichts droht bevorzugt dann, wenn ein Nutzer ungewollt stürzt. Ein Verlust des Gleichgewichts bezeichnet einen momentanen Zustand und ist nicht gleichzusetzen mit einem Verlust des Gleichgewichtssinns an sich. Stürze geschehen oftmals, wenn eine Person nicht mehr in der Lage ist, der auf den Körper wirkenden Schwerkraft eine kontrollierte Bewegung entgegenzusetzen und unfreiwillig sowie unsanft aus einer stehenden, gehenden, liegenden und/oder sitzenden Position auf den Boden fällt. Beispiele hierfür sind ein Stolpern oder ein Ausrutschen. Eine kontrollierte Bewegung wird vom zentralen Nervensystem bevorzugt aufgrund vieler verschiedener Sinneswahrnehmungen koordiniert. Der Gleichgewichtssinn nimmt hierbei typischerweise eine zentrale Bedeutung ein. Kommt es zu einer unkontrollierten Bewegung, liegt oft eine Fehleinschätzung zugrunde, welche auf einer gestörten Wahrnehmung, z. B. auf einem gestörten Gleichgewichtssinn beruht. Solche Störungen betreffen auch Gesunde, jedoch insbesondere Personen, die aufgrund eines Unfalls oder einer Krankheit an einer Störung des Gleichgewichts leiden, z. B. Schlaganfallpatienten. Eine Vorrichtung zur Sturzprävention stellt somit eine Vorrichtung dar, die durch technische Komponenten einem Nutzer bei der Haltung einer kontrollierten Gleichgewichtsposition unterstützen und vor drohenden Stürzen bewahren und warnen soll. Dafür muss die Vorrichtung u. a. mindestens einen Sensor umfassen, welche eine Körperposition und/oder eine Körperbewegung anhand relevanter Daten erfassen kann. Relevante Daten umfassen dabei die Geschwindigkeit der Bewegung, die Änderung der Geschwindigkeit der Bewegung (Beschleunigung) und/oder sogar die Änderung der Beschleunigung der Bewegung (Ruck). Auch Drehbeschleunigungen sind dabei bevorzugt zu beachten. Diese Daten müssen mindestens von einer Regelungseinheit verarbeitet und ausgewertet werden, wobei die Auswertung ergeben soll, ob ein Sturz droht oder nicht. Ist eine solche Gefahr gegeben, ist eine sofortige Warnung des Nutzers durch ein eindeutiges Signal eines Signalgebers erforderlich, welches den Nutzer zu einer Ausgleichs- oder Abfangbewegung zur Vermeidung des Sturzes veranlasst. Eine solche Vorrichtung kann somit bei Menschen mit bspw. krankheitsbedingten Gleichgewichtsstörungen behilflich sein, im Alltag ohne Stürze zurecht zu kommen. Ebenso kann die Vorrichtung durch das erzeugte Feedback einem Träger dabei helfen, wieder ein "Gefühl" für das Gleichgewicht zu entwickeln und eignet sich somit ebenfalls zu Therapiezwecken. Auch gesunde Träger können eine solche Vorrichtung zum Training neuer und schwieriger Bewegungsabläufe verwenden, z. B. Sportler oder Soldaten.

Eine Regelungseinheit ist geeignet zur Steuerung der Vorrichtung. Dabei kann die Regelungseinheit die Eingangssignale einlesen und Ausgangssignale erzeugen, wobei der Plural sich sowohl auf die Möglichkeit mehrerer zeitlich aufeinanderfolgender Signale als auch mehrere gleichzeitige Signale beziehen kann. In einer Mindestkonfiguration umfassen die Eingangssignale bevorzugt das Signal des Eingabemoduls und das Signal mindestens einen Sensors und die Ausgangssignale bevorzugt einen Signalgeber. Dabei kann eine Regelungseinheit z. B. anhand der Eingangssignale und/oder zusätzlich auf Basis gespeicherter Daten zu jedem Zeitpunkt ein Ausgangssignal erzeugen. Dieses kann bspw. binär sein in dem Sinne, das zu einem Zeitpunkt der Signalgeber entweder ein Feedbacksignal erzeugt oder nicht. Typischerweise "entscheidet" die Regelungseinheit über das Ausgangssignal als Funktion der Eingangssignale und/oder gespeicherter Signale anhand eines Algorithmus. Dieser kann entweder elektronisch in einer Hardware verdrahtet sein oder als Software programmiert sein, die z. B. durch einen geeigneten Prozessor ausgeführt wird. Eine Regelungseinheit ist bevorzugt eine integrierte Schaltung, besonders bevorzugt eine Digitalschaltung. Als Beispiele seien ein Computerchip, ein Mikroprozessor, ein Field Programmable Gate Array (FPGA) oder eine andere Datenverarbeitungsvorrichtung genannt. Eine Regelungseinheit kann bevorzugt Eingangs- und/oder Ausgangsdaten im Wesentlichen in Echtzeit verarbeiten.

Mit Messdaten sind insbesondere solche Daten oder Informationen gemeint, welche durch Messgeräte in Form des mindestens einen Sensors aufgenommen werden können. Ein Sensor kann z.B. ein Beschleunigungsmesser (synonym: Accelerometer) und/oder ein Gyrometer umfassen. Diese können bei der Erfüllung ihrer Funktionalität bspw. einen (zeitlichen) Strom digitaler Datensignale erzeugen, welche bevorzugt Eingangssignale der Regelungseinheit stellen.

Besonders bevorzugt betreffen die Messdaten Informationen über den Träger selbst, z.B. über Körperschwankungen des Trägers. Bevorzugt findet eine Erhebung der Messdaten durch den mindestens einen Sensor sowie eine Auswertung der Daten durch die Regelungseinheit automatisiert, d.h. bevorzugt ohne einen Input durch den Träger statt, solange die Vorrichtung eingeschaltet ist.

Bevorzugt können an die Regelungseinheit nicht nur Messdaten übertragen werden, sondern auch durch diese bearbeitet werden. Dazu kann neben mindestens einem Signaleingang bevorzugt ebenso über einen (Zwischen-) Speicher für die Messdaten verfügen.

Die Messdaten umfassen bevorzugt Daten über die Veränderung einer Körperposition und/oder des Bewegungsmusters des Trägers der Vorrichtung. Bevorzugt werden alle relevanten kinematischen Freiheitsgrade erfasst. Dabei können z. B. lineare Beschleunigungen entlang bevorzugt drei zueinander orthogonaler Raumrichtungen gemessen werden und/oder Drehbeschleunigungen um bevorzugt drei die Raumrichtungen aufspannender Achsen. Bevorzugt ist die Kenntnis aller sechs vorstehend genannten Beschleunigungen für eine umfassende Bestimmung der Position sowie einer Veränderung der Position erforderlich. Es kann aber bevorzugt sein, nur ausgewählte Linear- und/oder Drehbeschleunigungen zu messen, um eine effektive Sturzprävention zu ermöglichen. Als Beispiel sei eine Messung der Beschleunigung entlang der Longitudinalachse einer stehenden Person genannt. Wird bei dieser Messung eine starke Beschleunigung gemessen, kann dies bevorzugt als starkes Indiz für einen Sturz der Person gewertet werden. Ein Fachmann weiß, wie er anhand mathematischer Operationen, wie z. B. Integration, aus der Messung der Beschleunigungen entlang bevorzugt verschiedener Richtungen eine umfassende Information über die Position und/oder Bewegung eines vermessenen Körpers erhält. Die Vorrichtung wird bevorzugt am Körper getragen, so dass die aktuelle Körperposition bevorzugt im Wesentlichen durch die Position der Vorrichtung angenähert beschrieben werden kann. Durch geeignete Sensoren kann die Vorrichtung dabei die eigene Beschleunigung messen. Aus den Messungen wird die Veränderung der Körperposition und/oder des Bewegungsmusters bestimmt. Dabei können die Messdaten bspw. als Rohdaten einzelner, unabhängige Messungen verschiedener Messgeräte/Sensoren zu verschiedenen Zeitpunkten erzeugt und in der Regelungseinheit prozessiert werden, wobei sich die Veränderung der Körperposition und/oder des Bewegungsmusters erst durch die Prozessierung ergibt. Es kann ebenso bevorzugt sein, dass die Messgeräte z. B. in integrierter Form in einem Sensor vorliegen und die an die Regelungseinheit übermittelten Daten bereits eine Auswertung über die Veränderung der Körperposition und/oder des Bewegungsmusters umfassen. Bevorzugt umfassen die Messdaten einen mehrdimensionalen Vektor.

Die Veränderung der Körperposition und/oder des Bewegungsmusters des Trägers kann bevorzugt dreidimensional im Raum als Veränderung der Winkelgeschwindigkeit und/oder der Beschleunigung von Vorwärts-, Rückwärts- und Seitwärtsbewegungen des Körperschwerpunktes am Träger bestimmt werden. Mehrere Versuche weisen darauf hin, dass eine derartige Bestimmung der Veränderung der Körperposition und/oder des Bewegungsmusters besonders realistisch die Körperschwankung und/oder die Körperbewegungen beschreiben, die gesteigert auftreten, wenn der Gleichgewichtssinn einer Person gestört ist. Insbesondere dienen die Veränderung der Winkelgeschwindigkeit und/oder der Beschleunigung von Vorwärts-, Rückwärts- und Seitwärtsbewegungen des Körperschwerpunktes des Trägers als aussagekräftige Parameter zur Unterscheidung von willkürlichen Bewegungen und solchen Bewegungen, die durch Gleichgewichtsstörungen verursacht werden.

Als Sensor wird mindestens ein geeignetes Messgerät genannt, durch das eine Veränderung der Körperposition und/oder des Bewegungsmusters des Trägers der Vorrichtung erfasst werden kann. Bevorzugt sind mehrere Sensoren, also mehrere Messgeräte umfasst, insbesondere eine geeignete Anzahl Beschleunigungsmesser und/oder Gyrometer für eine lineare und/oder Drehratenbeschleunigung. Dabei kann der Sensor/können die Sensoren bevorzugt als kompakte Einheit umfassend alle vorhandenen Beschleunigungsmesser realisiert sein. Dabei kann bevorzugt ein einziges Ausgangsignal erzeugt werden, dass bereits eine Auswertung der Veränderung der Körperposition und/oder des Bewegungsmusters in einem für die Regelungseinheit geeignetem Datenformat enthält. Daher kann der mindestens eine Sensor zur Vornahme dieser Auswertung auch geeignete integrierte Schaltungen enthalten.

Die Vorrichtung wird bevorzugt an der Hüfte, nahe dem Körperschwerpunkt, getragen. Dadurch kann eine Position, Bewegung und/oder Verlagerung des Körperschwerpunktes im Wesentlichen bestimmt oder angenähert werden.

In einer besonders bevorzugten Ausführungsform geschieht dies mit Hilfe eines Gürtels, welcher das Gerät zur Messung der Veränderung der Körperposition und/oder des Bewegungsmusters in der Nähe der Hüfte arretiert. In weiteren bevorzugten Ausführungsformen erfolgt die Befestigung mit einem Gummiband oder mit einem gummifaserhaltigen Textilband erfolgt. Die Befestigung am Körper kann selbstverständlich auch über ein Ledergeschirr, Textilgeschirr oder über ein Synthetikledergeschirr erfolgen.

Vorteilhafterweise entspricht die gemessene Veränderung der Körperposition und/oder des Bewegungsmusters besonders genau der Veränderung der Position des Körperschwerpunktes des Trägers, wenn eine Positionierung des Messgerätes in der Nähe der Hüfte erfolgt. Insbesondere hat sich gezeigt, dass eine derart bestimmte Veränderung der Körperposition und/oder des Bewegungsmusters sehr realistisch Schwankungen und ggf. Stürze detektiert. Weiterhin wird durch eine derartige Positionierung des Messgerätes besonders gut eine kontrollierte Bewegung von einer unkontrollierten Bewegung unterschieden, welche bei Trägern der Vorrichtung mit einer Gleichgewichtsstörung verstärkt auftreten.

Die Vorrichtung kann jedoch auch an anderen Körperteilen getragen werden oder mit Sensoren und/oder Signalgebern Daten austauschen, die sich an weiteren Körperteilen befinden. So kann insbesondere auch durch die Zusammenführung der Daten verschiedener Messpunkte ein möglicher Sturz besonders frühzeitig erkannt und an besonders geeigneten Körperstellen das Feedback ausgelöst werden.

Es kann ebenso bevorzugt sein, dass die Vorrichtung am und/oder im Ohr getragen wird, z. B. in Form eines Kopfhörers, Brillenbügels etc. Ein Tragen der Vorrichtung am und/oder im Ohr ermöglicht eine besonders einfache, schnelle und stromsparende Auslösung eines akustischen Feedbacksignals, welche darüber hinaus nur sehr kurze Übertragungswege von Signalen benötigt, wodurch ein sehr schnelles Feedbacksignal erzeugt werden kann. Auch die Implementierung der Vorrichtung in technische Hörhilfen (z. B. Hörgeräte, Cochlea-Implantate) stellt eine bevorzugte Ausführungsvariante dar. Eine in ein Hörgerät und/oder Cochlea-Implantat integrierte Vorrichtung ist besonders praktisch, da häufig Gleichgewichtsstörungen und Hörprobleme nebeneinander auftreten.

Weiterhin stellt die Integration der Vorrichtung in eine Sehhilfe (z. B. Brille) eine bevorzugte Variante dar. Auch diese Ausführungsform ermöglicht die besonders einfache, schnelle und stromsparende Auslösung eines Feedbacksignals (z. B. visuell, vibrotaktil und/oder akustisch) mit sehr kurzen Signalwegen für ein besonders schnelles Feedback. Ein Feedback kann dabei bevorzugt visuell z. B. über eine Projektion eines optischen Feedbacksignals auf die Innenseite von Brillengläsern erfolgen.

Gerade die Erkennung von individuell ungewöhnlichen Bewegungsmustern, die zu einer Instabilität führen können, kann mithilfe mehrerer Sensoren verbessert werden. Bewegungsmuster ergeben sich aus den fortlaufenden Messwerten der Sensoren in allen Raumrichtungen. Durch die Ähnlichkeitsbeziehungen zwischen bereits gespeicherten, als instabil bewerteten Bewegungsmustern (hinsichtlich z. B. Richtung, Geschwindigkeit und Auslenkung) und dem aktuell gemessenen Bewegungsmuster kann eine sturznahe Situation erkannt werden. Beim Erkennen einer ausreichenden Ähnlichkeit wird der Träger der Vorrichtung durch ein Signal vor dem drohenden Sturz gewarnt. Die Vorgehensweise zur Erkennung der Ähnlichkeit von Bewegungsmustern ist dem Fachmann bekannt, z. B. mit Hilfe von Korrelationsanalyse(n) und/oder durch Maschinelles Lernen (engl.: *machine learning*)*.* Dabei kann das maschinelle Lernen im bevorzugt im Wesentlichen durch die Regelungseinheit der Vorrichtung selber vorgenommen werden für eine besonders leistungsstarke Vorrichtung und/oder zumindest teilweise durch eine regelmäßige Verbindung mit mindestens einem externen Computer vorgenommen werden, so dass die Anforderungen an die Rechenleistung der Regelvorrichtung überschaubar bleiben und diese kompakt und einfach realisiert werden kann.

Die Vorrichtung umfasst des Weiteren ein Eingabemodul. Ein Eingabemodul erlaubt dem Träger der Vorrichtung bevorzugt die Eingabe einer Information, welche durch die Regelungseinheit erfasst und ausgewertet werden kann. Das kann durch einen Knopf, einen Schalter, ein Touchscreen, eine Folientastatur, ein Mikrophon etc. realisiert werden. Im einfachsten Fall ist eine einzugebende Information binär und kann eine Antwort auf eine Ja/Nein-Frage sein. Das Eingabemodul muss bevorzugt konfiguriert sein, dem Träger zu signalisieren, dass eine Eingabe abgegeben werden soll.

Des Weiteren umfasst die Vorrichtung mindestens einen Signalgeber. Ein Signalgeber ist bevorzugt geeignet, eine von der Regelungseinheit generierte Information an den Träger der Vorrichtung zu übermitteln. Der Signalgeber hat die Funktion, einen Träger vor einem drohenden Sturzereignis zu warnen und so eine erhöhte Wachsamkeit und/oder eine schnelle Reaktion, z. B. zum Abfangen eines Sturzes zu auszulösen. Daher sollte die bevorzugt ablaufende Kette: Messung - Auswertung - Ansteuerung Signalgeber bevorzugt so schnell ablaufen, dass der Träger zeitlich die Möglichkeit hat, einen Sturz abzuwenden.

Das ausgegebene Signal des Signalgebers kann binär sein, z. B. als einfaches Warnsignal realisiert sein. Es kann jedoch auch ein komplexeres Signal ausgegeben werden, dass z. B. Information darüber enthält, in welche Richtung ein möglicher Sturz erfolgen könnte bzw. welche Gliedmaßen zum Abfangen aktiviert werden müssen.

Ein Signalgeber soll den Träger informieren und/oder involvieren. Daher sollte der Signalgeber auf mindestens einen Wahrnehmungssinn des Trägers gerichtet sein. Auch mehrere Sinne gleichzeitig können angesprochen werden. Angesprochen werden können bspw. der Tastsinn über vibrierende Aktoren bzw. Stimulatoren, das Sehen durch ein optisches Signal und/oder der Hörsinn über ein akustisches Signal. Es können aber auch durch Elektroden Muskelbewegungen oder nervliche Empfindungen ausgelöst werden, die dem Träger behilflich sein können, einen Sturz abzuwenden.

Die Regelungseinheit der Vorrichtung ist konfiguriert, in einem Betriebsmodus mittels des Signalgebers ein Feedbacksignal zu erzeugen, falls die gemessene Veränderung einer Körperposition und/oder des Bewegungsmusters eine Stabilitätsgrenze überschreitet, wobei die Stabilitätsgrenze ausgehend von einer Grundeinstellung auf Basis der Messdaten und der Eingabedaten kontinuierlich aktualisiert werden kann. Das Grundprinzip der Vorrichtung, aufgrund gemessener Daten eine Sturzgefahr zu evaluieren und bei akuter Gefahr ein Feedbacksignal zur Warnung des Trägers auszugeben, wurde vorstehend bereits erklärt. Nun soll die Bedeutung der Stabilitätsgrenze näher erläutert werden. Diese Stabilitätsgrenze bezeichnet bevorzugt mindestens einen Wert bzw. ein Ensemble an Werten, welche eine Schwelle darstellen. Messdaten, welche diese in der Stabilitätsgrenze festgelegten Werte überschreiten, werden als Indikation einer Sturzgefahr angesehen, wodurch die Regelungseinheit die Erzeugung eines Feedbacksignals veranlasst. Die Stabilitätsgrenze kann bevorzugt viele verschiedene Formen annehmen und mehrdimensional sein. Das Ensemble könnte z. B. in einem einfachen, illustrativen Beispiel aus Schwelle von Beschleunigungswerten in zueinander orthogonalen Raumrichtungen x, y und z bestehen. Dabei könnte jeder einzelne Wert eine Stabilitätsgrenze darstellen, bei deren Überschreitung ein Sturz vermutet wird. Es könnte jedoch ebenso bevorzugt sein, dass mindestens zwei Werte gemeinsam eine Stabilitätsgrenze bilden, deren Überschreiten ein Feedbacksignal auslöst. Auch könnte eine geeignete Funktion diese Werte abbilden, wobei für den Wert der Abbildung eine Stabilitätsgrenze formuliert ist. Es können ebenso komplexere, höherdimensionale Werte-Tupel zur Bestimmung der Stabilitätsgrenze herangezogen werden, welche z. B. neben Beschleunigungswerten auch Geschwindigkeiten und/oder Positionen umfasst. Auch können Daten zu unterschiedlichen Messzeitpunkten für die Grenze herangezogen werden. Ein Fachmann weiß, dass zur Bestimmung einer Sturzgefahr verschiedene Werte herangezogen werden können, für die eine Stabilitätsgrenze formuliert wird. Dies kann sich nach verschiedenen Faktoren richten und sich z. B. nach der bevorzugten Anwendung oder einer Abwägung aus Funktionalität und Komplexität bestimmen. Bevorzugt umfasst die Stabilitätsgrenze einen mehrdimensionalen Vektor. Dabei kann dieser Vektor bevorzugt dasselbe Format haben (sich auf die gleichen Grö-ßen) beziehen wie die bevorzugten Messdaten in Vektorform. Bevorzugt umfasst die Stabilitätsgrenze ein Vektorfeld mit vielen einzelnen Vektoren, welche eine einzelne Stabilitätsgrenze repräsentieren. Diese bilden bevorzugt eine geschlossene Oberfläche im Vektorraum. Bevorzugt gibt ein direkter Vergleich zwischen den bevorzugten Daten in Vektorform und dem Vektor bzw. Vektorfeld der Stabilitätsgrenze an, ob diese überschritten wurde. Z. B. kann überprüft werden, ob ein Messdatenvektor innerhalb oder außerhalb der geschlossenen Oberfläche im Vektorraum der Stabilitätsgrenze liegt.

Diese Stabilitätsgrenze ist bevorzugt ab Werk auf der Vorrichtung gespeichert und/oder kann vorab z. B. aus verschiedenen Tests als sinnvolle Grenze bestimmt worden sein. Diese Stabilitätsgrenze ist bevorzugt die Grundeinstellung.

Ein Abgleich von (ausgewerteten) Messdaten mit der Stabilitätsgrenze durch die Regelungseinheit ergibt dabei bevorzugt, ob durch die Regelungseinheit ein Feedbacksignal zur Sturzprävention ausgegeben wird.

Die Stabilitätsgrenze ist jedoch aktualisierbar und unterliegt bevorzugt Anpassungen aufgrund von Veränderungen der Körperposition und/oder des Bewegungsmusters des Trägers, welche die Stabilitätsgrenze überschreiten, bei denen jedoch kein Sturz des Trägers stattgefunden hat.

Eine ab Werk festgesetzte Stabilitätsgrenze bzw. Grundeinstellung ist vorteilhafterweise so ausgelegt, dass unabhängig vom jeweiligen Träger und seiner körperlichen Konstitution jeglicher Sturz vermieden werden kann. So soll z. B. auch ein Träger im fortgeschrittenen Alter mit langsamen Bewegungen und eingeschränktem Gleichgewichtssinn vor Stürzen gewarnt werden. Deshalb ist ab Werk eine "konservativ" ausgelegte Stabilitätsgrenze typisch. Diese Grenze kann jedoch bspw. bei einem jüngeren und agilen Träger, welcher sich schnell bewegt, dazu führen, dass ständig ein Feedbacksignal zur Warnung erzeugt wird, ohne dass die Bewegungen tatsächlich unkontrolliert sind oder eine Sturzgefahr besteht. Dies führt in der Regel zu einer schlechten Compliance des Trägers, weil dieser durch die Signale genervt ist und die Vorrichtung seltener trägt und/oder er sich an die Signale gewöhnt und diese nicht mehr als Warnung wahrnimmt, mithin die Vigilanz des Trägers vermindert wird. Dies kann dazu führen, dass der Träger bei einer echten Gefahr das Feedbacksignal ignoriert und stürzt. Die "Kunst" einer effektiven Vorrichtung mit hoher Compliance besteht darin, rechtzeitig einen Sturz zu erkennen, ohne (häufig) einen Fehlalarm auszulösen und somit die Vigilanz auf einem hohen Niveau zu halten. Daher wird bevorzugt bei Überschreiten einer Stabilitätsgrenze nicht nur ein Feedbacksignal ausgelöst, sondern der Träger wird zu einer Eingabe am Eingabemodul veranlasst, bzw. der Träger nimmt diese von sich aus vor. Dabei umfasst die Eingabe bevorzugt eine Information darüber, ob tatsächlich ein Sturz stattgefunden hat. Hat kein Sturz stattgefunden, wird die Stabilitätsgrenze bevorzugt anhand der Messdaten, welche die Grenze überschritten haben, aktualisiert. In einem einfachen Fall kann die Stabilitätsgrenze z. B. direkt aus diesen Messwerten bestehen oder aus ihnen gebildet werden.

So wird bei einem leichten Überschreiten der ursprünglichen Stabilitätsgrenze, welche jedoch unterhalb einer aktualisierten, neuen Grenze liegt, bevorzugt kein Feedbacksignal mehr ausgegeben. Stabilitätsgrenzen können bevorzugt an kontrollierte Bewegungen angepasst werden. So kann eine Art selbstlernender Algorithmus realisiert werden. Es hat sich gezeigt, dass Menschen mit unterschiedlicher körperlicher Gesundheit und mit unterschiedlichem Training sehr unterschiedliche, sinnvolle Stabilitätsgrenzen benötigen. Eine Bewegung, die für den einen kontrolliert ist oder aber nur einen Beinahe-Sturz bedeutet, ist für eine andere Position ein sicherer Indikator für einen Sturz.

Es ist jedoch wichtig zu betonen, dass ein "Beinahe-Sturz" sich bevorzugt auch auf unkontrollierte Bewegungen beziehen kann, bei der aber ein Sturz noch abgewandt werden konnte. Es hat sich herausgestellt, dass ähnliche Bewegungen zu einem früheren Faststurz, der gerade noch abgefangen werden konnte, ebenfalls abgefangen werden können, da diese Abfangreaktion bevorzugt eine feststehende Fähigkeit eines Trägers ist, die immer wieder in ähnlichen, unkontrollierten Situationen angewendet werden kann. Somit ist es sehr vorteilhaft, die Stabilitätsgrenze dahingehend anzupassen, dass solche ähnlichen Bewegungen, welche für einen Träger abfangbare Faststürze darstellen, nicht durch ein Feedbacksignal angekündigt werden, um die Vigilanz des Trägers nicht zu verringern.

Es hat sich nämlich gezeigt, dass die zentralnervöse Verarbeitung der multimodalen sensorischen Informationen im Gleichgewichtssystem hinsichtlich der Verarbeitungsgeschwindigkeit und der Effektorintensität wesentlich von der aktuellen Aufmerksamkeit bzw. Vigilanz abhängt. So wird jeder Gleichgewichtsreflex bei hoher Vigilanz schneller und stärker ausgeführt als bei geringer Vigilanz. Eine hohe Vigilanz kann mithilfe einer kontinuierlichen Aktivierung aufrechterhalten bzw. durch geeignete plötzliche Reize erzeugt werden. Wird die Erhöhung der Vigilanz durch plötzliche Reize im richtigen Zeitpunkt erreicht, können stabilisierende Bewegungen zur Sturzverhinderung eingeleitet werden. Dabei ist es besonders bedeutsam, dass diese plötzlichen Reize nur bei der Abwendung eines Sturzereignisses zur Anwendung kommen, da bei zu häufiger Reizdarbietung eine Gewöhnung einsetzt und somit die gewünschte Erhöhung der Vigilanz ausbleibt. Somit soll der Reiz nur dann ausgelöst werden, wenn die Verlagerung des Körperschwerpunktes einen für die Stabilität der Körperhaltung kritischen Wert erreicht. Dieser Wert ist sehr individuell und kann von der erfindungsgemäßen Vorrichtung durch einen "selbstlernenden Algorithmus" wie vorstehend beschrieben bestimmt werden.

Dazu misst die Vorrichtung bzw. das Gerät bevorzugt fortlaufend die Körperschwankung nahe am Körperschwerpunkt (Hüfte). Dies kann in einem Ausführungsbeispiel mithilfe von Sensoren in Form von Gyrometern und/oder Accelerometern in 2 bis 3 Raumachsen geschehen. In dieser Ausführungsform wird bevorzugt nach einem Überschreiten der Stabilitätsgrenze der neue Maximalwert in bspw. einer Schwankungsrichtung im Gerät gespeichert und solange nicht überschrieben, bis ein höherer Wert ohne ein Sturz- oder fast-Sturzereignis in der entsprechenden Raumachse ermittelt wurde. Bevor ein Wert als neuer Maximalwert, bevorzugt in einer Schwankungsrichtung im Gerät gespeichert wird, interagiert das Gerät bevorzugt mit dem Anwender bzw. umgekehrt. Dieser bestätigt oder verneint das (Fast-) Sturzereignis. Wurde ein fast-Sturzereignis festgestellt, ist der Lernvorgang zunächst abgeschlossen. In einer bevorzugten Anwendung der Erfindung absolviert der Nutzer zur Durchführung einer individuellen Grundeinstellung des Gerätes einen Parcours mit sensomotorischen Konditionen des Alltags. Dabei kann bevorzugt der Signalgeber vorübergehend ausgeschaltet werden. Nach der erfolgten Grundeinstellung wird die Ausgabe des Zusatzreizes (Feedbacksignal) bevorzugt aktiviert, der die Vigilanz in fast-Sturzsituationen deutlich erhöhen soll. Bei jeder Überschreitung des Maximalwertes in einer Schwankungsrichtung interagiert das Gerät weiterhin mit dem Nutzer, bevorzugt, indem es eine Bestätigung oder Ablehnung eines fast-Sturzereignisses oder Sturzes verlangt. So kann eine möglichst exakte Übereinstimmung der Reizausgabe mit einem fast-Sturzereignis erreicht werden.

Das Gerät eignet sich also zur Prävention von Stürzen im Alltag durch Auslösen eines Feedbacksignals bei erhöhtem Sturzrisiko zur Verhinderung von Stürzen und weist ein selbstlernendes Verfahren zur fortwährenden Bestimmung des individuellen Sturzrisikos auf. Die Vorrichtung zur Sturzprävention des Menschen umfasst mindestens einen Sensor zur Messung der Verlagerung des Körperschwerpunktes und eine computergesteuerte Regelungseinheit, die basierend auf Messdaten und der Interaktion mit dem Anwender kritische Bereiche hinsichtlich der Körperstabilität erkennt und ein Feedbacksignal aussendet um durch die Erhöhung der Vigilanz eine Stabilisierung der Körperhaltung einzuleiten.

Die Balanceregelungsvorrichtung enthält bevorzugt Gyrometer und/oder Accelerometer, sowie einen Mikroprozessor, einen elektronischen Speicher, Elemente zur Interaktion mit dem Nutzer (z. B. Sprachausgabe, Tastatur, Bildschirm) und Treiber (z. B. Verstärker) für die Ausgabe des Feedbacksignals. Sie wird bevorzugt an der Hüfte, nahe dem Körperschwerpunkt, getragen. Als Feedbacksignal eignet sich besonders ein akustischer Stimulus (z. B. Reinton im Bereich von 20 Hz bis 20 kHz), ein visuelles Signal (z. B. Lichtblitz im Bereich von 380-780 nm) und/oder ein vibrotaktiles Signal (z. B. Vibration im Bereich von 5-1000 Hz).

In der erfindungsgemäßen Vorrichtung zur Sturzprävention aktualisiert die Regelungseinheit im Betriebsmodus die Stabilitätsgrenze, indem eine vorherige Stabilitätsgrenze auf eine gemessene Veränderung der Körperposition und/oder des Bewegungsmusters angepasst wird, falls die gemessene Veränderung der Körperposition und/oder des Bewegungsmusters die vorherige Stabilitätsgrenze überschreitet und am Eingabemodul bestätigt wird, dass kein Sturz stattgefunden hat. In dieser Ausführungsform gibt nach jedem Überschreiten einer Stabilitätsgrenze der Träger an, ob tatsächlich ein Sturz stattgefunden hat oder nicht. Hat ein Sturz stattgefunden, bleibt die Grenze unverändert. Hat jedoch kein Sturz trotz der Überschreitung stattgefunden, wird die Grenze angepasst. So kann die Vorrichtung individualisiert und an ein Bewegungsmuster bzw. die koordinativen Fähigkeiten des Trägers angepasst werden. Dabei wird die geschilderte Vorgehensweise bevorzugt nur im sogenannten Betriebsmodus ausgeführt. Dieser gibt bevorzugt an, ob die Vorrichtung grundsätzlich eingeschaltet ist und sich nicht im bevorzugt ebenfalls einstellbaren Lernmodus befindet. Bevorzugt kann eine Eingabe dabei über mindestens eine Schnittstelle, z. B. mindestens einen Schalter und/oder ein Touchscreen, aber auch akustisch über ein Mikrofon kombiniert mit einer Spracherkennung erfolgen. Beispielsweise können bevorzugt an der Vorrichtung zwei Schalter vorliegen, von denen der eine bedeutet, dass ein Sturz stattgefunden hat ("Sturz ja") und der andere, dass kein Sturz stattgefunden hat ("Sturz nein"). Durch die bevorzugte Verwendung von zwei separaten Schaltern bzw. Eingabemodulen, welche darüber hinaus durch verschiedene Farb- und/oder Formgebung gut voneinander unterscheidbar sind, können Fehleingaben vermieden werden, insbesondere in einem Moment emotionaler Erregung aufgrund eines (Beinahe-) Sturzes.

In einer bevorzugten Ausführungsform öffnet sich nach der Eingabe, dass ein Sturz stattgefunden hat, bevorzugt ein Hilfedialog auf einer Anzeigevorrichtung und/oder über einen Lautsprecher der Vorrichtung. Durch diesen Hilfedialog, welcher mittels weiterer Eingabemodule (Schalter, Touchscreen, Mikrofon etc.) geführt werden kann, kann bevorzugt Hilfe angefordert werden, insbesondere über ein mit der Vorrichtung verbindbares Smartphone und/oder über ein eigenes Kommunikationsnetzwerk der Vorrichtung mit einer Rettungsstelle. Ebenso können bevorzugt Hilfestellungen gegeben werden, wie ein sicheres Aufstehen ermöglicht werden kann.

In einer weiteren bevorzugten Ausführungsform ist die Regelungseinheit im Betriebsmodus konfiguriert, ein Zurücksetzen der Stabilitätsgrenze auf die Grundeinstellung zu ermöglichen. So kann bspw. bei einem Wechsel des Trägers wieder auf die besonders sichere Grundeinstellung zurückgegriffen werden und eine neue Anpassung der Grenze für den neuen Träger vorgenommen werden. Es können auch bevorzugt mehrere Trägerprofile angelegt und eingestellt werden, die die jeweiligen Stabilitätsgrenzen abspeichern und abrufen. So kann eine Vorrichtung bevorzugt von mehreren Trägern mit unterschiedlichen Bewegungsfähigkeiten und/oder für verschiedene Anwendungen (z. B. Alltagsverrichtungen, Sport etc.) verwendet werden. Ebenso kann nicht ausgeschlossen werden, dass eine Veränderung der Stabilitätsgrenze, welche sich aus einem früheren Beinahe-Sturz ergeben hat, in einer neuen Situation vom Träger nicht mehr abgefangen werden kann. Dann ist es eventuell geboten, die Stabilitätsgrenze aus Sicherheitsgründen zurückzusetzen.

Es kann ebenso bevorzugt sein, dass in dem vorgenannten Fall ein Zurücksetzen stattfindet, welches nicht die Grundeinstellung betrifft, sondern die Grenze auf Zwischenwerte zurücksetzt, die in einer etwas höheren Sicherheit als die zuletzt eingestellte Stabilitätsgrenze resultiert. So kann ein besonders guter Kompromiss gefunden werden, der Sicherheit ohne unnötige Feedbacksignale generiert.

Es kann bevorzugt auch festgelegt werden, dass eine einmal gefundene Stabilitätsgrenze fest beibehalten wird, wenn sich diese als ideal erwiesen hat. Dann wird ein Träger bevorzugt auch bei einem Überschreiten der Stabilitätsgrenze nicht um eine Eingabe gebeten. So kann ein Träger entscheiden, ob er seine Stabilitätsgrenze weiter verschieben oder beibehalten will.

In einer weiteren bevorzugten Ausführungsform umfasst die Vorrichtung ein Ausgabemodul, welches bei Überschreiten der Stabilitätsgrenze mittels des Signalgebers den Träger auffordert einzugeben, ob ein Sturz stattgefunden hat. So kann bevorzugt erreicht werden, dass ein Träger über ein Überschreiten der Stabilitätsgrenze informiert wird, auch wenn z. B. kein Sturz stattgefunden hat. Es kann weiterhin bevorzugt verhindert werden, dass ein Träger eine Eingabe vergisst. Das erhöht die Compliance, also bevorzugt die aktive Einbeziehung und Bereitschaft der Träger zur aktiven Mitwirkung.

In einer weiteren bevorzugten Ausführungsform umfasst das Ausgabemodul einen Lautsprecher und/oder ein Display.

Durch Erzeugung eines Tones oder einer verbalen Aufforderung über einen Lautsprecher kann ein Träger besonders effektiv zu einer Eingabe auffordert. Dabei können insbesondere angenehme Töne bzw. eine angenehme Stimme zur verbalen Aufforderung eine hohe Compliance des Trägers bewirken.

Es kann ebenfalls bevorzugt ein Display zur Eingabeaufforderung durch das Ausgabemodul verwendet werden. Dieses kann bevorzugt zusätzlich aufleuchten und/oder farblich unterlegt sein. Ein Display ist insbesondere vorteilhaft, wenn es in Form eines Touchscreens realisiert ist, auf dem gleichzeitig die Eingabe getätigt werden kann. So kann eine Eingabe besonders intuitiv und effizient realisiert werden.

In einer bevorzugten Ausführungsform ist die Regelungseinheit konfiguriert, ein Feedbacksignal mittels des Signalgebers innerhalb einer Zeitspanne von 0 ms bis 500 ms zu erzeugen, nachdem auf Basis der Messdaten des mindestens einen Sensors ein Überschreiten der Stabilitätsgrenze festgestellt wurde. Damit das Feedback erfolgreich zur Prävention von Stürzen verwendet werden kann, darf zwischen einer festgestellten Überschreitung der Stabilitätsgrenze aufgrund der Messdaten und einem ausgegebenen Feedbacksignal nur eine sehr kurze Zeitspanne vergehen. Dabei ist zu beachten, dass in dieser Zeitspanne Messung der gefährlichen Körperposition, Auswertung und Feststellen eines Überschreitens der Grenze und Ausgabe des Feedbacksignals stattfinden muss und noch genug Zeit bleiben vorhanden bleibt, um eine Abfangreaktion des Trägers auszulösen.

Die vorstehend genannte Zeitspanne hat sich dabei als besonders geeignet erwiesen, bei einer durchschnittlichen oder unterdurchschnittlichen Reaktionszeit noch genügend zeitliche Reserven für eine ausreichende Reaktion zu lassen. Zur Realisierung dieser kurzen Zeitspanne ist bevorzugt die Verwendung einer schnellen Elektronik umfassend Sensor, Regelungseinheit und Signalgeber angezeigt, sondern auch eine Implementation kurzer elektronischer Signalwege zwischen den einzelnen Komponenten. Insbesondere eine Verwendung einer Regelungseinheit in Form einer integrierten Schaltung mit ausreichender Rechenleistung sowie geeignetem Speichermedium hat sich für eine kurze Zeitspanne als vorteilhaft erwiesen. Ebenso wird eine Optimierung eines Algorithmus der Regelungseinheit hierfür bevorzugt.

In einer bevorzugten Ausführungsform liegen Regelungseinheit, Sensor(en), Eingabemodul und Signalgeber in einem gemeinsamen Gehäuse installiert vor und sind über elektrische Signalleitungen miteinander verbunden. Dadurch können besonders einfach und effektiv vorstehend genannte Zeitspannen realisiert werden. Insbesondere eine Verwendung von elektrischen Signalleitungen, bevorzugt in Form von Kabeln und/oder Leiterbahnen, ist hierfür geeignet. Zwar bieten sich auch Signalübertragungen zwischen den Komponenten über drahtlose Verbindungen an, jedoch können diese Signale leichter gestört werden, wodurch längere Übertragungsraten entstehen können. Vorteilhafterweise ist der Aufbau dabei besonders kompakt gehalten, wodurch Signalwege räumlich und somit zeitlich besonders kurz sind. Besonders bevorzugt liegen alle wesentlichen Komponenten gemeinsam auf einer Leiterplatte installiert vor. Weiterhin sorgt das Gehäuse bevorzugt für eine gute Abschirmung der Elektronik, wodurch weniger Störungen verursacht werden und Signallaufzeiten besonders kurz sind.

In einer weiteren bevorzugten Ausführungsform ist der mindestens eine Signalgeber ausgesucht aus der Gruppe umfassend einen Lautsprecher, eine optische Anzeige und/oder ein Vibrationsstimulator.

Das Feedbacksignal wird dabei bevorzugt über den Lautsprecher, die optische Anzeige und/oder einen Vibrationsstimulator erzeugt und kann durch eine rechtzeitige Warnung vor einem Sturz zur Sturzprävention verwendet werden. Ein Lautsprecher ist bevorzugt in die Vorrichtung integriert und erzeugt einen Signalton. Ein Signalton hat sich für eine besonders effektive Warnung zur Sturzprävention als besonders geeignet erwiesen. Es hat sich gezeigt, dass Töne besonders gut von einem Träger aufgenommen werden und eine Gewöhnung an einen Signalton, welche eine verringerte Vigilanz zur Folge hätte, besonders selten ist. In einer besonders bevorzugten Variante wird die Lautstärke und/oder die Frequenz des akustischen Signales gesteigert je stärker die bestimmte Veränderung der Körperposition und/oder des Bewegungsmusters von der Stabilitätsgrenze abweicht.

Das Feedbacksignal kann (ebenso wie die Eingabeaufforderung) über eine Sprachausgabe erfolgen. Diese kann aus psychologischen Gründen als besonders angenehm und/oder wirksam empfunden werden und erhöht die Compliance.

Das Feedbacksignal kann (ebenso wie die Eingabeaufforderung) über ein Headset bzw. Hörgerät erfolgen. Gleichgewichtsstörungen gehen oft mit Störungen des Hörsinns einher. Daher kann es sinnvoll sein, das Feedbacksignal über ein Hörgerät auszugeben. Dabei kann eine Verbindung zwischen Hörgerät und Vorrichtung per Kabel und/oder drahtlos sein oder aber die Vorrichtung im Hörgerät und/oder Headset integriert sein.

Eine optische Anzeige ist bevorzugt eine Anzeigevorrichtung und/oder ein Display, auf der ein Warnsignal optisch, z. B. durch Darstellung eines Symbols (Warndreieck o. ä.) dargestellt und zur Sturzprävention verwendet werden kann. Dabei kann die Anzeige zusätzlich farblich gestaltet sein und/oder zur Warnung aufleuchten, bspw. in Signalrot. Die Anzeige ist dabei bevorzugt innerhalb des Blickfeldes des Trägers angebracht. Eine optische Anzeige kann auch projiziert werden, z. B. auf die Innenseite von Brillengläsern einer (Sonnen-) Brille. Optische Signale, welche durch eine Anzeige vermittelt werden, rufen eine besonders schnelle Reaktion hervor, wodurch das Sturzrisiko weiter vermindert werden kann.

Eine optische Anzeige kann ebenso bevorzugt eine Lichtquelle sein, bevorzugt eine Lichtquelle im Blickfeld des Trägers der Bestrahlungsvorrichtung, so dass bei Aktivität der Aktoren der Träger einen Lichtreiz wahrnimmt. Die Intensität des Lichtreizes oder aber die Farbe des Lichtreizes wird dabei in einer bevorzugten Variante, derart gewählt, dass diese eine höhere Signalwirkung aufweist, je stärker die bestimmte Veränderung der Körperposition und/oder des Bewegungsmusters von der Stabilitätsgrenze abweicht.

Ein Vibrationsstimulator löst bevorzugt eine mechanische Vibration aus, die auf einen Bereich der Körperoberfläche, bevorzugt auf die Haut eines Trägers übertragen werden kann. Der Stimulator kann dabei fest am Körper des Trägers befestigt sein, z. B. mit einem Band, einem Clip an der Hüfte oder einem Gürtel. Ebenso kann ein solcher Stimulator in eine Kleidung integriert sein. Ein Vibrationsstimulator löst auf besonders intuitive Weise eine Abfangreaktion eines Trägers zur Sturzprävention aus. Insbesondere können auch mehrere Simulatoren verwendet werden, welche so an mehreren Körperstellen eines Trägers befestigt werden, dass durch eine Vibration eines geeigneten Stimulators von einem Träger direkt auf die geeignete Abfangreaktion geschlossen werden kann. Z. B. können an der Hüfte vier Stimulatoren befestigt werden, welche gleichmäßig um die Hüfte verteilt werden. Dabei kann jeder vibrierende Stimulator einen Quadranten im Raum symbolisieren, in dessen Richtung eine akute Sturzgefahr festgestellt wurde. Bspw. kann bei einer Sturzgefahr in die vom Träger aus gesehene linke, vordere Richtung bevorzugt ein vorderer und ein seitlich links an der Hüfte befestigter Stimulator eine Vibration auslösen.

Bevorzugte Aktoren sind z.B. Unruhemotor 6CH-1201-WL-00, Namiko Corp., Tokyo. Die Drehzahl des Unruhemotors ist hierbei bevorzugt abhängig von der Frequenz der ausgegebenen Impulse. Die Zahl der Impulse kann dabei bevorzugt an die Größe der Sturzgefahr angepasst, werden, welche sich z. B. durch Größe der Überschreitung der Stabilitätsgrenze bemessen kann. So kann eine besonders effektive und angepasste Warnung des Trägers vor Stürzen realisiert werden.

Weitere bevorzugte Aktoren sind galvanische Stimulatoren, wobei die Stimulatoren durch elektrische Reizung auf der Körperoberfläche, durch elektrische Reizung von motorischen Nerven bzw. der Muskulatur und/oder durch elektrische Reizung von sensorischen Nerven bzw. Sinnesorganen oder Teilen dieser ausgeführt wird. Diese können besonders effektiv eine Reaktion des Trägers auslösen.

In einer weiteren bevorzugten Ausführungsform wird das Feedbacksignal über Zeitdauer von 100 ms bis 5000 ms erzeugt. Es hat sich gezeigt, dass ein Feedbacksignal bevorzugt eine gewisse Mindestdauer haben sollte, damit es zu einer bewussten Wahrnehmung kommt. Gleichzeitig hat sich herausgestellt, dass ein Signal nicht zu lange dauern sollte, damit eine prägnante, impulsartige Wirkung erhalten bleibt, die eine besonders wirksame Warnfunktion transportiert und somit die Vigilanz, die Sicherheit und die Effizienz erhöht.

In einer bevorzugten Ausführungsform ist der Signalgeber ein Lautsprecher und das Feedbacksignal ein akustisches Signal mit einer Frequenz von 20 Hz bis 20 kHz, besonders bevorzugt 50 Hz bis 18 kHz und einer Lautstärke von 40 dB bis 120 dB SPL, besonders bevorzugt 50 dB bis 100 dB SPL. SPL steht bevorzugt für Schalldruckpegel. Diese Werte haben sich als besonders wirksam erwiesen und sind vom menschlichen Gehör besonders gut erfassbar. Gleichzeitig kann innerhalb der Bereiche eine Variation der akustischen Frequenz und/oder der Lautstärke vorgenommen werden, welche verschiedene Abstufungen von Sturzgefahren aufgrund unterschiedlicher Überschreitungen der Stabilitätsgrenzen repräsentieren können.

In einer weiteren bevorzugten Ausführungsform ist der Signalgeber eine optische Anzeige und das Feedbacksignal ein visuelles Signal mit einer Wellenlänge von 380 - 780 nm. Signale dieser Wellenlänge sind besonders gut für das Auge sichtbar, gleichzeitig kann innerhalb des Spektrum die Farbe des optischen Feedbacks variiert werden, um z. B. verschiedene Abstufungen von Sturzgefahren aufgrund unterschiedlicher Überschreitungen der Stabilitätsgrenzen repräsentieren zu können.

In einer bevorzugten Ausführungsform ist der Signalgeber ein Vibrationsstimulator und das Feedbacksignal ein vibrotaktiles Signal mit einer Frequenz von 0,1 bis 1000 Hz, besonders bevorzugt 3 bis 800 Hz. Diese Frequenzen sind besonders gut vom menschlichen Tastsinn wahrnehmbar und erlauben eine Variation der Feedbackfrequenz, welche bevorzugt an die Größe der Sturzgefahr angepasst werden, die sich z. B. durch Größe der Überschreitung der Stabilitätsgrenze bemessen kann.

In einer weiteren bevorzugten Ausführungsform ist die Regelungseinheit konfiguriert, in einem Lernmodus die Grundeinstellung der Stabilitätsgrenze zu erlernen, wobei die Stabilitätsgrenze kontinuierlich auf eine gemessene Veränderung der Körperposition und/oder des Bewegungsmusters angepasst wird, sobald die Veränderung der Körperposition und/oder des Bewegungsmusters eine vorherige Stabilitätsgrenze überschreitet.

Dieser Lernmodus ist bevorzugt zu unterscheiden von einem Betriebsmodus. In einem Lernmodus kann der Träger der Vorrichtung z. B., bevorzugt unter Aufsicht von geschultem Personal, Übungen z. B. einen Parcours zu absolvieren, um sinnvolle Grundeinstellungen der Stabilitätsgrenze zu definieren. Dabei muss er bevorzugt nicht ständig bei einer Überschreitung einer Stabilitätsgrenze über das Eingabemodul bestätigen, dass kein Sturz stattgefunden hat. Die Stabilitätsgrenze wird bevorzugt ohne eine solche Eingabe angepasst, sobald eine abgespeicherte Grenze überschritten wird. Dies kann gerade anfangs während dieser Lernphase häufig der Fall sein. Die Lernphase umfasst bevorzugt gezielt bestimmte Bewegungen des Trägers, die eine starke Veränderung der Körperposition und/oder des Bewegungsmusters mit sich bringen, ohne dass jedoch tatsächlich ein Sturz droht. Insbesondere geht es dabei um bestimmte, unvermeidliche Alltagsbewegungen des Trägers wie z. B. Bücken, Setzen, Treppen steigen o. ä. Müsste dabei jedes Mal bestätigt werden, dass kein Sturz stattgefunden hat, wäre dies sehr ineffektiv. Grundeinstellungen sind bevorzugt individuell auf eine Person abgestimmte Stabilitätsgrenzen, die einem Betriebsmodus anfänglich zu Grunde gelegt werde und die bevorzugt alltägliche Bewegungen des Individuums erlauben, ohne dass ständig die Grenzen überschritten werden, wodurch fälschlicherweise eine Sturzgefahr durch die Vorrichtung erkannt würde und ein Feedback veranlasst würde. Auch bei einer neuen Vorrichtung müssen von Anfang an Stabilitätsgrenzen gegeben sein, mit denen die Messdaten verglichen werden, um die Sturzgefahr zu bemessen. Diese können bei einem geeigneten Anfangswert liegen, der z. B. "Null" ist und somit jegliche Bewegung zunächst zu einer Überschreitung der Grenze führt. Ebenso könnte das Gerät ab Werk bereits Stabilitätsgrenzen aufweisen, die sich z. B. aus Versuchsreihen als sinnvoll erwiesen haben. Jedoch sind diese Stabilitätsgrenzen oft aus Sicherheitsgrenzen so "niedrig" angelegt, dass es anfangs im Betriebsmodus zu permanenter Warnung vor Stürzen kommen könnte. Es ist in jedem Fall vorteilhaft, anfangs diese Grundeinstellung zu erlernen bzw. anzupassen. Insbesondere ist es sinnvoll, individuelle Grundeinstellung zu erlernen, da die Fähigkeiten verschiedener Träger der Vorrichtung sich stark unterscheiden können, je nach Alter, allgemeinem Gesundheitszustand und spezifischem Leiden der Person. Eine für eine Person geeignete Stabilitätsgrenze kann für eine andere Person nur einen stark eingeschränkten Bewegungsspielraum ohne Erzeugung eines Feedbacksignals bedeuten.

Zum Erlernen einer Grundeinstellung kann bevorzugt ein standardisierter Balancetest durchgeführt werden, z. B. der Standard Balance Deficit Test (SBDT). Der SBDT ist ein standardisierter Balancetest in dem der Träger der Vorrichtung verschiedene Übungen durchgeführt. Insbesondere umfasst der Balancetest
bevorzugt folgende Übungen:
- Stehen auf zwei Beinen mit offenen Augen
- Stehen auf zwei Beinen mit geschlossenen Augen
- Stehen auf einem Bein mit offenen Augen
- Stehen auf einem Bein mit geschlossenen Augen
- Acht Tandem Schritte (einen Fuß vor den anderen) mit offenen Augen
- Stehen auf zwei Beinen auf Schaumstoff (Höhe 10 cm, Dichte 25 kg / m3) sowohl mit offenen als auch geschlossen Augen
- Stehen auf einem Bein auf Schaumstoff
- Acht Tandem Schritte auf Schaumstoff
- 3 m gehen während der Kopf kreisend bewegt wird
- 3 m gehen während der Kopf im Rhythmus abwechselnd nach links und rechts gedreht wird
- 3 m gehen während mit dem Kopf im Rhythmus genickt wird
- 3 m mit geschlossenen Augen gehen
- Über vier Barrieren gehen, wobei die Barrieren eine Höhe von 26 cm aufweisen und zwischen den Barrieren jeweils ein Abstand von 1 m besteht
- Hinsetzen und aufstehen

Durch solche oder ähnliche, gezielte und standardisierte Übungen zum Erlernen der Grundeinstellung können besonders effektiv sinnvolle Stabilitätsgrenzen erlernt werden, die sich an alltäglichen Bewegungen orientieren, bei denen keine Sturzgefahr besteht.

In einer bevorzugten Ausführungsform ist die Regelungseinheit konfiguriert, in einem Lernmodus bei Überschreiten der Stabilitätsgrenze mittels des Signalgebers kein Feedbacksignal auszugeben.

Es kann somit bevorzugt im Lernmodus nicht nur auf eine Eingabe verzichtet werden, sondern ebenso kann ein Feedback unterbleiben. Es kann sinnvoll sein, dieses Feedbacksignal zu unterdrücken, wenn beim Erlernen der Grundeinstellung besonders häufig eine Stabilitätsgrenze überschritten wird und somit ein ebenso häufiges Feedbacksignal erzeugt wird. Im Lernmodus hat das Feedback ohnehin eher informativen Charakter für den Träger, in dem es anzeigt, dass eine neue Bewegung außerhalb der alten Grenze liegt und besitzt bevorzugt keine Warnfunktion. In bestimmten Fällen und z. B. je nach Vorliebe des Trägers kann es vorteilhaft sein, auf diese informative Funktion zu verzichten, um keinen Gewöhnungseffekt beim Träger zu erzeugen, welcher eine Compliance verschlechtern könnte

In einer weiteren bevorzugten Ausführungsform umfasst die Regelungseinheit einen Mikroprozessor und/oder einen elektronischen Speicher.

Durch solch eine Regelungseinheit kann die Vorrichtung besonders einfach und günstig realisiert werden. Ein elektronischer Speicher kann Daten, z. B. zur Stabilitätsgrenze, besonders schnell aufrufen und verarbeiten bzw. abspeichern.

Besonders bevorzugt wird als Regelungseinheit ein *ATmega32U4* verwendet. Dieser hat sich als besonders geeignet erwiesen und stellt einen guten Kompromiss aus schneller Prozessierung und günstigen Kosten sowie einfacher Programmierung dar. Insbesondere in einer effizienten und kostengünstigen Variante in Verbindung mit einer Sprachausgabe (Schallgeber bzw. Lautsprecher) und eine Folientastatur für den Nutzerinput (Eingabemodul) ist diese Regelungseinheit vorteilhaft.

In einer weiteren bevorzugten Ausführungsform umfasst der mindestens eine Sensor zur Messung der Veränderung einer Körperposition und/oder des Bewegungsmusters des Trägers der Vorrichtung Gyrometer und/oder Beschleunigungssensoren.

Diese haben sich als Sensor zur Messung der Veränderung einer Körperposition und/oder des Bewegungsmusters des Trägers als besonders sinnvoll erwiesen. Es kann bevorzugt sein, dass hier kommerziell erhältliche Gyrometer und/oder Beschleunigungssensoren verwendet werden, die kostengünstig sind und ihre Tauglichkeit bereits bei einer Vielzahl von Anwendungen unter Beweis gestellt haben, somit besonders robust und wartungsarm sind. Es kann diesbezüglich je nach Verwendung und Anforderungen auf verschiedene Technologien zurückgegriffen werden, die unterschiedliche Vorteile aufweisen. Es können als Gyrometer z. B. hochpräzise Sagnac-Interferometer verwendet werden. Aber auch der Einsatz günstiger und robuster MEMS-Technologie für Gyrometer und/oder Beschleunigungssensoren kann bevorzugt werden.

Dabei können standardisierte Komponenten, die ein standardisiertes Datenausgabeformat aufweisen, bevorzugt sein. Diese können besonders einfach und schnell in der Herstellung mit einer Regelungseinheit funktionell im Sinne der Erfindung verknüpft werden.

Auch integrierte Komponenten, welche die geeignete Anzahl an Gyrometern und/oder Beschleunigungssensoren integriert in einer Schaltung aufweisen, können bevorzugt sein für eine besonders kompakte und schnelle Vorrichtung.

In einer weiteren bevorzugten Ausführungsform wird die Veränderung einer Körperposition und/oder des Bewegungsmusters des Trägers der Vorrichtung dreidimensional im Raum als Veränderung der Winkelgeschwindigkeit und/oder der Beschleunigung von Vorwärts-, Rückwärts- und Seitwärtsbewegungen des Körperschwerpunktes am Träger bestimmt.

Eine Veränderung einer Körperposition und/oder des Bewegungsmusters des Trägers der Vorrichtung kann bevorzugt durch die (lineare) Beschleunigung des Körperschwerpunktes in drei Raumdimensionen und die Veränderung der Winkelgeschwindigkeit des Körperschwerpunktes in drei Raumdimensionen beschrieben werden. Die Beschleunigung von Vorwärts-, Rückwärts- und Seitwärtsbewegungen meint bevorzugt die lineare Beschleunigung in drei Raumdimensionen, welche vorteilhafterweise durch senkrecht aufeinander stehende Körperachsen, bevorzugt durch den Körperschwerpunkt, insbesondere durch Longitudinalachse, Transversalachse und Sagittalachse, aufgespannt werden. Anhand dieser Achsen lässt sich bevorzugt eine Vorwärts-, Rückwärts- und/oder Seitwärtsbewegung beschreiben. Auch sind diese Achsen bevorzugt geeignet, Winkelgeschwindigkeiten und deren Veränderungen zu beschreiben, indem bevorzugt eine Körperdrehung anhand von Drehungen um diese Achsen beschrieben wird. Durch diese Ausführungsform lässt sich eine Veränderung einer Körperposition und/oder eines Bewegungsmusters besonders präzise und gleichzeitig effizient beschrieben. So kann auch Rechenleistung der Regelungseinheit gespart werden, insbesondere, wenn die gleichen Achsen für die Vorwärts-, Rückwärts- und Seitwärtsbewegungen und die Veränderung der Winkelgeschwindigkeit verwendet werden.

In einer bevorzugten Ausführungsform weist der mindestens eine Sensor zur Messung der Veränderung einer Körperposition und/oder des Bewegungsmusters des Trägers mehrere orthogonal zueinanderstehende Gyrometer und/oder Beschleunigungssensoren auf, welche die Veränderung der Winkelgeschwindigkeit und/oder die Beschleunigung von Vorwärts-, Rückwärts- und Seitwärtsbewegungen des Körpers bestimmen.

Durch mehrere, orthogonal zueinanderstehende Gyrometer und/oder Beschleunigungssensoren können Veränderungen der Winkelgeschwindigkeit und/oder der Beschleunigung von Vorwärts-, Rückwärts- und Seitwärtsbewegungen des Körpers verbessert festgestellt werden. Bevorzugt bilden die orthogonal zueinanderstehenden Gyrometer und/oder Beschleunigungssensoren bezüglich ihrer Orientierung die entsprechenden Achsen nach, bevorzugt Longitudinalachse, Transversalachse und Sagittalachse. Dadurch ist die Messung besonders effizient und schnell, auch aufgrund eingesparter Rechenleistung.

In einer bevorzugten Ausführungsform wird die Veränderung der Winkelgeschwindigkeit von Vorwärts-, Rückwärts- und Seitwärtsbewegungen des Körperschwerpunktes in 3 Dimensionen dabei von einem 3-Achsen-Gyrometer-Chip bestimmt. Der 3-Achsen-Gyrometer-Chip umfasst dabei bevorzugt drei Gyrometer, welche orthogonal zueinander angeordnet sind. Ein besonders bevorzugter 3-Achsen-Gyrometer-Chip ist der L3G4200D von STMicroelectronics. Gyrometer sind dabei bevorzugt als Geräte zu verstehen, welche Drehbewegungen insbesondere Winkelgeschwindigkeiten der Drehbewegungen messen können. Geeignete Gyrometer sind dem Fachmann bekannt und können kommerziell erworben werden. Insbesondere sind im Stand der Technik Gyrometer bekannt, welche die Corioliskraft, als Trägheitskraft in rotierenden Bezugsystemen, bestimmen. Dabei werden in diesen Gyrometern abhängig von der Veränderung der Corioliskraft während einer Bewegung Kapazitätsveränderungen registriert. Da die Corioliskraft in einer festen Beziehung zur Winkelgeschwindigkeit der Rotation steht, können die gemessenen Kapazitätsveränderungen mithilfe eines Mikroprozessors in numerische Werte der Winkelgeschwindigkeit umgewandelt werden. Ebenso können 3-Achsen-Gyrometer-Chips kommerziell erworben werden. Durch die orthogonale Anordnung von drei Gyrometern in den 3-Achsen-Gyrometer-Chips, kann die Winkelgeschwindigkeit einer Drehung des Gerätes bevorzugt zur Messung der Veränderung einer Körperposition und/oder des Bewegungsmusters in 3 Dimensionen, d.h. entlang von drei Bezugsachsen, bestimmt werden.

In einer weiteren Ausführungsform umfasst das Gerät zur Messung der Veränderung einer Körperposition und/oder des Bewegungsmusters zwei Gyrometer, welche orthogonal zueinander angeordnet sind. Diese beiden Gyrometer bestimmen die Winkelgeschwindigkeit in zwei orthogonalen Bezugsachsen. Vorteilhafterweise kann durch die Berechnung der Winkelgeschwindigkeit in der dritten orthogonalen Bezugsachse aus den anderen, bekannten Winkelgeschwindigkeiten entlang der x- oder y-Achse, ein Gyrometer gespart werden. D.h. in dieser Ausführungsform werden statt drei Gyrometern wie bei dem 3-Achsen-Gyrometer nur zwei Gyrometer verwandt. Dies führt insbesondere zu einer leichteren Konstruktion des Gerätes zur Bestimmung der Veränderung der Körperposition und/oder des Bewegungsmusters und ist besonders kosteneffizient.

In bevorzugten Varianten sind an dem Gerät zur Messung der Veränderung der Körperposition und/oder des Bewegungsmusters Programme wählbar, welche dem Gerät anzeigen, welche Tätigkeit der Träger der Vorrichtung ausübt oder ausüben will. Die Programmwahl kann dabei bevorzugt manuell oder über eine Spracherkennung erfolgen. Weiterhin kann das Gerät die Programme auch selbständig aktivieren, indem beispielsweise Bewegungsabläufe mithilfe der Auswertung von Hirn- oder Muskelaktivitäten erkannt werden. So kann z. B. eine je nach Anwendung angepasste Stabilitätsgrenze bzw. erlernte Grundeinstellung verwendet werden.

### Detaillierte Beschreibung

Im Folgenden soll die Erfindung an Hand von Beispielen und Figuren näher erläutert werden, ohne auf diese beschränkt zu sein.

### Kurzbeschreibung der Abbildungen

**Figur 1** zeigt eine schematische Darstellung der Vorrichtung.
**Figur 2** zeigt eine schematische Darstellung des Erlernens der Stabilitätsgrenze im Lernmodus.
**Figur 3** zeigt eine schematische Darstellung des IR des Erlernens der Stabilitätsgrenze im Betriebsmodus.

### Detaillierte Beschreibung der Abbildung

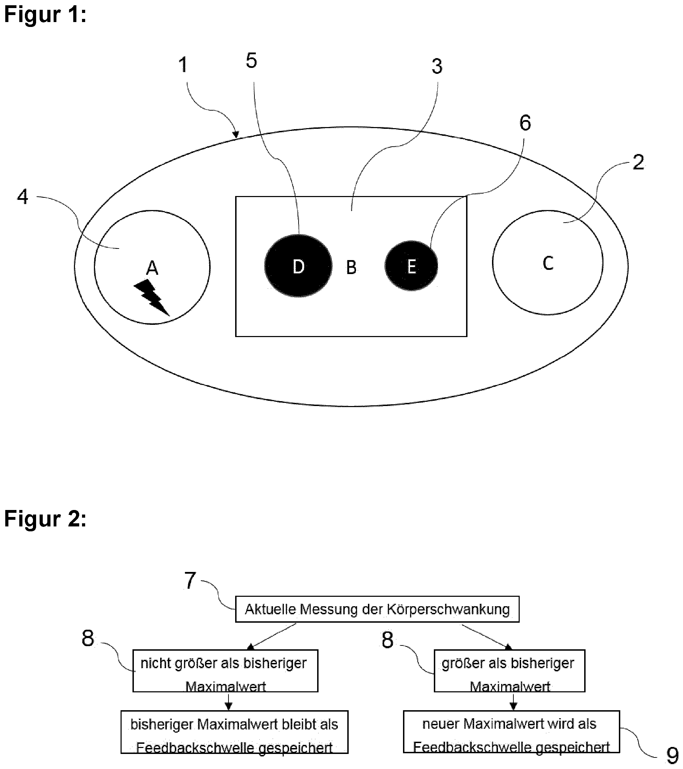

Figur 1 zeigt eine schematische Darstellung der Vorrichtung **1,** die durch einen Träger getragen wird. Die Vorrichtung **1** weist Sensoren **2** in Form beispielsweise von Beschleunigungssensoren und/oder (dreiachsigen) Gyrometern auf. Diese sind mit einer Regelungseinheit **3** verbunden, die bei Überschreiten einer Stabilitätsgrenze einen Signalgeber **4** zu einem Feedback veranlasst, bspw. über Aktuatoren, die ein vibrotaktiles Feedback auslösen. Die Vorrichtung umfasst des Weiteren Eingabemodule **5, 6** über die beim Überschreiten der Stabilitätsgrenze eingegeben werden kann, ob ein Sturz stattgefunden hat (Eingabemodul "Sturz ja" **5**) oder nicht (Eingabemodul "Sturz nein" **6**). Somit wird im zweiten Fall im Betriebsmodus die Stabilitätsgrenze verschoben **9,** da die Änderung der Körperposition und/oder des Bewegungsmusters des Trägers keinem Sturz entsprach.

Figur 2 zeigt eine schematische Darstellung des Erlernens der Stabilitätsgrenze im Lernmodus. In diesem Modus wird ohne Abfrage über ein Sturzereignis jede gemessene Veränderung einer Körperposition oder eines Bewegungsmusters **7** für eine Aktualisierung der Stabilitätsgrenze **9** herangezogen, wenn bei einem Abgleich der Stabilitätsgrenze **8** festgestellt wird, dass diese überschritten wurde. Dabei wird bevorzugt im Lernmodus kein Feedbacksignal **10** durch den Signalgeber **4** ausgegeben. In diesem Lernmodus kann besonders schnell und einfach eine sinnvolle Stabilitätsgrenze ermittelt werden. Bevorzugt werden im Lernmodus bestimmte Übungen oder Alltagsbewegungen gezielt vom Träger absolviert.

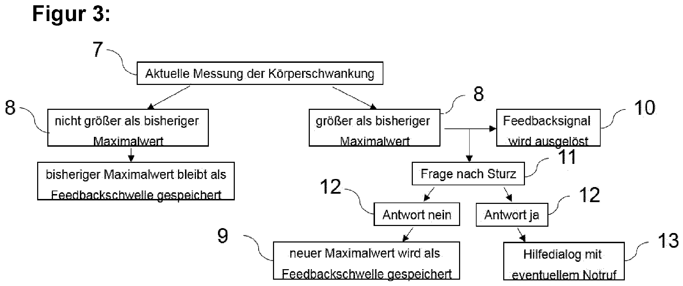

Figur 3 zeigt eine schematische Darstellung des Erlernens der Stabilitätsgrenze im Betriebsmodus. In diesem wird eine gemessene Veränderung einer Körperposition oder eines Bewegungsmusters **7** wie im Lernmodus zunächst einmal einem Abgleich mit der Stabilitätsgrenze **8** unterzogen. Wird diese überschritten, wird zunächst ein warnendes Feedbacksignal **10** durch den Signalgeber **4** ausgegeben. Dann wird eine Eingabeaufforderung **11** vorgenommen, nach der der Träger über die Eingabe **12** das Stattfinden eines Sturzes bestätigen oder verneinen muss. Im ersten Fall, wenn ein Sturz stattgefunden hat, öffnet sich bevorzugt ein Hilfedialog **13** und es wird ggf. ein Notruf an eine Rettungsstelle versendet. Im zweiten Fall, wenn es keinen Sturz gab, wird eine Aktualisierung der Stabilitätsgrenze **9** vorgenommen.

Es wird darauf hingewiesen, dass verschiedene Alternativen zu den beschriebenen Ausführungsformen der Erfindung verwendet werden können, um die Erfindung auszuführen und zu der erfindungsgemäßen Lösung zu gelangen. Die erfindungsgemäße Vorrichtung beschränkt sich in ihren Ausführungen somit nicht auf die vorstehenden bevorzugten Ausführungsformen. Vielmehr ist eine Vielzahl von Ausgestaltungsvarianten denkbar, welche von der dargestellten Lösung abweichen können. Ziel der Ansprüche ist es, den Schutzumfang der Erfindung zu definieren. Der Schutzumfang der Ansprüche ist darauf gerichtet, die erfindungsgemäße Vorrichtung sowie äquivalente Ausführungsformen von diesen abzudecken.

### Bezugszeichenliste

- 1: Vorrichtung zur Sturzprävention
- 2: Sensor
- 3: Regelungseinheit
- 4: Signalgeber
- 5: Eingabemodul "Sturz ja"
- 6: Eingabemodul "Sturz nein"
- 7: Gemessene Veränderung einer Körperposition oder eines Bewegungsmusters
- 8: Abgleich Stabilitätsgrenze
- 9: Aktualisierung der Stabilitätsgrenze
- 10: Feedbacksignal
- 11: Eingabeaufforderung
- 12: Eingabe
- 13: Hilfedialog

## Patentansprüche

1. Vorrichtung zur Sturzprävention (1) umfassend
a) eine Regelungseinheit (3)
b) mindestens einen Sensor (2) zur Messung der Veränderung einer Körperposition und/oder eines Bewegungsmusters des Trägers der Vorrichtung (1), welcher Messdaten an die Regelungseinheit (3) überträgt, wobei die Veränderung einer Körperposition und/oder des Bewegungsmusters (7) des Trägers der Vorrichtung (1) dreidimensional im Raum als Veränderung der Winkelgeschwindigkeit und/oder der Beschleunigung von Vorwärts-, Rückwärts- und Seitwärtsbewegungen des Körperschwerpunktes am Träger bestimmt wird
c) ein Eingabemodul (5, 6), welches Eingabedaten an die Regelungseinheit (3) überträgt
d) mindestens einen Signalgeber (4), welcher auf mindestens einen Wahrnehmungssinn des Trägers gerichtet ist
wobei die Regelungseinheit (3) konfiguriert ist in einem Betriebsmodus mittels des Signalgebers (4) ein Feedbacksignal (10) an den Träger der Vorrichtung innerhalb eines Intervalls zwischen 0 und 500 ms zu erzeugen, falls die gemessene Veränderung einer Körperposition und/oder eines Bewegungsmusters (7) eine Stabilitätsgrenze überschreitet, wobei die Stabilitätsgrenze ausgehend von einer Grundeinstellung auf Basis der Messdaten und der Eingabedaten kontinuierlich aktualisiert (9) werden kann, indem eine vorherige Stabilitätsgrenze auf eine gemessenen Veränderung der Körperposition angepasst wird, falls die gemessenen Veränderung der Körperposition und/oder des Bewegungsmusters (7) die vorherige Stabilitätsgrenze überschreitet und am Eingabemodul (5; 6) bestätigt wird, dass kein Sturz stattgefunden hat.

2. Vorrichtung zur Sturzprävention (1) gemäß einem oder mehreren der vorherigen Ansprüche
**dadurch gekennzeichnet, dass**
die Regelungseinheit (3) im Betriebsmodus konfiguriert ist ein Zurücksetzen der Stabilitätsgrenze auf die Grundeinstellung zu ermöglichen.

3. Vorrichtung zur Sturzprävention (1) gemäß einem oder mehreren der vorherigen Ansprüche
**dadurch gekennzeichnet, dass**
die Vorrichtung (1) ein Ausgabemodul umfasst, welches bei Überschreiten der Stabilitätsgrenze mittels des Signalgebers den Träger auffordert einzugeben (11), ob ein Sturz stattgefunden hat.

4. Vorrichtung zur Sturzprävention (1) gemäß dem vorherigen Anspruch
**dadurch gekennzeichnet, dass**
das Ausgabemodul einen Lautsprecher und/oder ein Display umfasst.

5. Vorrichtung zur Sturzprävention (1) gemäß einem oder mehreren der vorherigen Ansprüche
**dadurch gekennzeichnet, dass**
der mindestens eine Signalgeber (4) ausgesucht ist aus der Gruppe umfassend einen Lautsprecher, eine optische Anzeige und/oder ein Vibrationsstimulator.

6. Vorrichtung zur Sturzprävention (1) gemäß einem oder mehreren der vorherigen Ansprüche
**dadurch gekennzeichnet, dass**
das Feedbacksignal (10) über eine Zeitdauer von 100 ms bis 5000 ms erzeugt wird.

7. Vorrichtung zur Sturzprävention (1) gemäß einem oder mehreren der vorherigen Ansprüche
**dadurch gekennzeichnet, dass**
der Signalgeber (4) ein Lautsprecher ist und das Feedbacksignal (10) ein akustisches Signal mit einer Frequenz von 20 Hz bis 20 kHz, besonders bevorzugt 50 Hz bis 18 kHz und einer Lautstärke von 40 dB bis 120 dB SPL, besonders bevorzugt 50 dB bis 100 dB SPL.

8. Vorrichtung zur Sturzprävention (1) gemäß einem oder mehreren der vorherigen Ansprüche
**dadurch gekennzeichnet, dass**
der Signalgeber (4) eine optische Anzeige ist und das Feedbacksignal (10) ein visuelles Signal mit einer Wellenlänge von 380 - 780 nm.

9. Vorrichtung zur Sturzprävention (1) gemäß einem oder mehreren der vorherigen Ansprüche
**dadurch gekennzeichnet, dass**
der Signalgeber (4) ein Vibrationsstimulator ist und das Feedbacksignal (10) ein vibrotaktiles Signal mit einer Frequenz von 0,1 bis 1000 Hz, besonders bevorzugt 3 bis 800 Hz.

10. Vorrichtung zur Sturzprävention (1) gemäß einem oder mehreren der vorherigen Ansprüche
**dadurch gekennzeichnet, dass**
die Regelungseinheit (3) konfiguriert ist in einem Lernmodus die Grundeinstellung der Stabilitätsgrenze zu erlernen, wobei die Stabilitätsgrenze kontinuierlich auf eine gemessene Veränderung der Körperposition und/oder des Bewegungsmusters angepasst wird (9), sobald die Veränderung der Körperposition und/oder des Bewegungsmusters (7) eine vorherige Stabilitätsgrenze überschreitet.

11. Vorrichtung zur Sturzprävention (1) gemäß einem oder mehreren der vorherigen Ansprüche
**dadurch gekennzeichnet, dass**
die Regelungseinheit (3) konfiguriert ist in einem Lernmodus bei Überschreiten der Stabilitätsgrenze mittels des Signalgebers (4) kein Feedbacksignal (10) auszugeben.

12. Vorrichtung zur Sturzprävention (1) gemäß einem oder mehreren der vorherigen Ansprüche
**dadurch gekennzeichnet, dass**
Regelungseinheit (3), Sensor (2), Eingabemodul (5, 6) und Signalgeber (4) in einem gemeinsamen Gehäuse installiert vorliegen und über elektrische Signalleitungen miteinander verbunden sind.

13. Vorrichtung zur Sturzprävention (1) gemäß einem oder mehreren der vorherigen Ansprüche
**dadurch gekennzeichnet, dass**
die Regelungseinheit (3) einen Mikroprozessor und/oder einen elektronischen Speicher umfasst.

14. Vorrichtung zur Sturzprävention (1) gemäß einem oder mehreren der vorherigen Ansprüche
**dadurch gekennzeichnet, dass**
der mindestens eine Sensor (2) zur Messung der Veränderung einer Körperposition und/oder des Bewegungsmusters (7) des Trägers der Vorrichtung (1) Gyrometer und/oder Beschleunigungssensoren umfasst.

15. Vorrichtung zur Sturzprävention (1) gemäß einem oder mehreren der vorherigen Ansprüche
**dadurch gekennzeichnet, dass**
der mindestens eine Sensor (2) zur Messung der Veränderung einer Körperposition und/oder des Bewegungsmusters (7) des Trägers mehrere orthogonal zueinanderstehende Gyrometer und/oder Beschleunigungssensoren aufweist, welche die Veränderung der Winkelgeschwindigkeit und/oder die Beschleunigung von Vorwärts-, Rückwärts- und Seitwärtsbewegungen des Körpers bestimmen.

## Claims

1. A fall prevention device (1), comprising
a) a control unit (3)
b) at least one sensor (2) for measuring the change in a body position and/or a movement pattern of the wearer of the device (1), which transmits measurement data to the control unit (3), the change in a body position and/or the movement pattern (7) of the wearer of the device (1) being determined three-dimensionally in space as a change in the angular velocity and/or the acceleration of forward, backward and sideways movements of the body's center of gravity on the wearer;
c) an input module (5, 6) which transmits input data to the control unit (3);
d) at least one signal transmitter (4) which is directed towards at least one of the wearer's senses of perception;
wherein the control unit (3) is configured in an operating mode to generate a feedback signal (10) to the wearer of the device within an interval of between 0 and 500 ms by means of the signal transmitter (4), if the measured change in a body position and/or a movement pattern (7) exceeds a stability limit, with the stability limit being able to be continuously updated (9), starting from a basic setting on the basis of the measurement data and the input data, by a previous stability limit being adapted to a measured change in the body position if the measured change in the body position and/or the movement pattern (7) exceeds the previous stability limit and it is confirmed on the input module (5; 6) that no fall has taken place.

2. The device for fall prevention (1) according to any one or more of the preceding claims,
**characterized in that**
the control unit (3) is configured in the operating mode to allow the stability limit to be reset to the basic setting.

3. The device for fall prevention (1) according to any one or more of the preceding claims,
**characterized in that**
the device (1) comprises an output module which, when the stability limit is exceeded, prompts the wearer to enter (11) whether a fall has occurred, by means of the signal transmitter.

4. The device for fall prevention (1) according to the previous claim,
**characterized in that**
the output module comprises a loudspeaker and/or a display.

5. The device for fall prevention (1) according to any one or more of the preceding claims,
**characterized in that**
the at least one signal transmitter (4) is selected from the group comprising a loudspeaker, an optical display and/or a vibration stimulator.

6. The device for fall prevention (1) according to any one or more of the preceding claims,
**characterized in that**
the feedback signal (10) is generated over a period of time of 100 ms to 5000 ms.

7. The device for fall prevention (1) according to any one or more of the preceding claims,
**characterized in that**
the signal transmitter (4) is a loudspeaker, and the feedback signal (10) is an acoustic signal with a frequency of 20 Hz to 20 kHz, particularly preferably 50 Hz to 18 kHz and a volume of 40 dB to 120 dB SPL, particularly preferably 50 dB to 100 dB SPL.

8. The device for fall prevention (1) according to any one or more of the preceding claims,
**characterized in that**
the signal transmitter (4) is an optical display, and the feedback signal (10) is a visual signal with a wavelength of 380 - 780 nm.

9. The device for fall prevention (1) according to any one or more of the preceding claims,
**characterized in that**
the signal transmitter (4) is a vibration stimulator, and the feedback signal (10) is a vibrotactile signal with a frequency of 0.1 to 1000 Hz, particularly preferably 3 to 800 Hz.

10. The device for fall prevention (1) according to any one or more of the preceding claims,
**characterized in that**
the control unit (3) is configured to learn the basic setting of the stability limit in a learning mode, with the stability limit being continuously adapted to a measured change in the body position and/or the movement pattern (9) as soon as the change in the body position and/or the movement pattern (7) exceeds a previous stability limit.

11. The device for fall prevention (1) according to any one or more of the preceding claims,
**characterized in that**
the control unit (3) is configured in a learning mode not to output a feedback signal (10) by means of the signal transmitter (4) when the stability limit is exceeded.

12. The device for fall prevention (1) according to any one or more of the preceding claims,
**characterized in that**
control unit (3), sensor (2), input module (5, 6) and signal transmitter (4) are installed in a common housing and are connected to one another via electrical signal lines.

13. The device for fall prevention (1) according to any one or more of the preceding claims,
**characterized in that**
the control unit (3) comprises a microprocessor and/or an electronic memory.

14. The device for fall prevention (1) according to any one or more of the preceding claims,
**characterized in that**
the at least one sensor (2) for measuring the change in a body position and/or the movement pattern (7) of the wearer of the device (1) comprises gyrometers and/or acceleration sensors.

15. The device for fall prevention (1) according to any one or more of the preceding claims,
**characterized in that**
the at least one sensor (2) for measuring the change in a body position and/or the movement pattern (7) of the wearer has several orthogonal gyrometers and/or acceleration sensors which determine the change in angular velocity and/or the acceleration of forward, backward and sideways movements of the body.

## Revendications

1. Dispositif de prévention de chute (1) comprenant
a) une unité de commande (3)
b) au moins un capteur (2) pour mesurer le changement d'une position corporelle et/ou d'un modèle de mouvement du porteur du dispositif (1), qui transmet des données de mesure à l'unité de commande (3), dans lequel le changement d'une position corporelle et/ou du modèle de mouvement (7) du porteur du dispositif (1) est déterminé en trois dimensions dans l'espace comme un changement de la vitesse angulaire et/ou l'accélération des mouvements avant, arrière et latéraux du centre de gravité du corps sur le porteur
c) un module d'entrée (5, 6) qui transmet des données d'entrée à l'unité de commande (3)
d) au moins un émetteur de signal (4) qui est dirigé vers au moins un des sens de perception du porteur
dans lequel l'unité de commande (3) est configurée dans un mode de fonctionnement pour utiliser l'émetteur de signal (4) pour générer un signal de retour (10) au porteur du dispositif dans un intervalle compris entre 0 et 500 ms si le changement mesuré d'une position corporelle et/ou d'un modèle de mouvement (7) dépasse une limite de stabilité, dans lequel la limite de stabilité peut être mise à jour en continu (9), à partir d'un réglage de base sur la base des données de mesure et des données d'entrée, dans lequel une limite de stabilité précédente est adaptée à un changement mesuré de la position corporelle si le changement mesuré de la position corporelle et/ou du modèle de mouvement (7) dépasse la limite de stabilité précédente et s'il est confirmé sur le module d'entrée (5 ; 6) qu'aucune chute n'a eu lieu.

2. Dispositif de prévention de chute (1) selon une ou plusieurs des revendications précédentes
**caractérisé en ce que**
l'unité de commande (3) est configurée en mode marche pour permettre la réinitialisation de la limite de stabilité au réglage par défaut.

3. Dispositif de prévention de chute (1) selon une ou plusieurs des revendications précédentes
**caractérisé en ce que**
le dispositif (1) comprend un module de sortie qui, lorsque la limite de stabilité est dépassée, utilise l'émetteur de signal pour demander au porteur de saisir (11) si une chute à eu lieu.

4. Dispositif de prévention de chute (1) selon la revendication précédente
**caractérisé en ce que**
le module de sortie comprend un haut-parleur et/ou un affichage.

5. Dispositif de prévention de chute (1) selon une ou plusieurs des revendications précédentes
**caractérisé en ce que**
l'au moins un émetteur de signal (4) est choisi dans le groupe comprenant un haut-parleur, un affichage optique et/ou un stimulateur de vibrations.

6. Dispositif de prévention de chute (1) selon une ou plusieurs des revendications précédentes
**caractérisé en ce que**
le signal de retour (10) est généré sur une période de 100 ms à 5000 ms.

7. Dispositif de prévention de chute (1) selon une ou plusieurs des revendications précédentes
**caractérisé en ce que**
l'émetteur de signal (4) est un haut-parleur et le signal de retour (10) est un signal acoustique avec une fréquence de 20 Hz à 20 kHz, de manière particulièrement préférée de 50 Hz à 18 kHz et un volume de 40 dB à 120 dB SPL, de manière particulièrement préférée de 50 dB à 100 dB SPL.

8. Dispositif de prévention de chute (1) selon une ou plusieurs des revendications précédentes
**caractérisé en ce que**
le générateur de signal (4) est un affichage optique et le signal de retour (10) est un signal visuel avec une longueur d'onde de 380 à 780 nm.

9. Dispositif de prévention de chute (1) selon une ou plusieurs des revendications précédentes
**caractérisé en ce que**
l'émetteur de signal (4) est un stimulateur de vibration et le signal de retour (10) est un signal vibrotactile avec une fréquence de 0,1 à 1000 Hz, de manière particulièrement préférée de 3 à 800 Hz.

10. Dispositif de prévention de chute (1) selon une ou plusieurs des revendications précédentes
**caractérisé en ce que**
l'unité de commande (3) est configurée pour apprendre le réglage de base de la limite de stabilité dans un mode d'apprentissage, dans lequel la limite de stabilité est adaptée en continu à un changement mesuré de la position corporelle et/ou du modèle de mouvement (9) dès que le changement de la position corporelle et/ou du modèle de mouvement (7) dépasse une limite de stabilité précédente.

11. Dispositif de prévention de chute (1) selon une ou plusieurs des revendications précédentes
**caractérisé en ce que**
l'unité de commande (3) est configurée dans un mode d'apprentissage pour ne pas émettre de signal de retour (10) au moyen de l'émetteur de signal (4) lorsque la limite de stabilité est dépassée.

12. Dispositif de prévention de chute (1) selon une ou plusieurs des revendications précédentes
**caractérisé en ce que**
L'unité de commande (3), le capteur (2), le module d'entrée (5, 6) et l'émetteur de signal (4) sont installés dans un boîtier commun et sont reliés les uns aux autres par des lignes de signaux électriques.

13. Dispositif de prévention de chute (1) selon une ou plusieurs des revendications précédentes
**caractérisé en ce que**
l'unité de commande (3) comprend un microprocesseur et/ou une mémoire électronique.

14. Dispositif de prévention de chute (1) selon une ou plusieurs des revendications précédentes
**caractérisé en ce que**
l'au moins un capteur (2) pour mesurer le changement d'une position corporelle et/ou du modèle de mouvement (7) du porteur du dispositif (1) comprend des gyromètres et/ou des capteurs d'accélération.

15. Dispositif de prévention de chute (1) selon une ou plusieurs des revendications précédentes
**caractérisé en ce que**
l'au moins un capteur (2) pour mesurer le changement d'une position corporelle et/ou le modèle de mouvement (7) du porteur comporte plusieurs gyromètres et/ou capteurs d'accélération orthogonaux qui déterminent le changement de vitesse angulaire et/ou l'accélération des mouvements vers l'avant, vers l'arrière et latéraux du corps.
